(19) Europäisches Patentamt
European Patent Office
Office européen des brevets

(11) Veröffentlichungsnummer: 0 288 867
A2

# (12) EUROPÄISCHE PATENTANMELDUNG

(21) Anmeldenummer: 88106182.4

(22) Anmeldetag: 19.04.88

(51) Int. Cl.4: C07C 91/40 , C07D 263/14 , C07D 263/06 , C07F 7/18 , C07D 307/14 , C07D 309/14 , C07C 93/26 , A23K 1/16

(30) Priorität: 30.04.87 DE 3714484

(43) Veröffentlichungstag der Anmeldung: 02.11.88 Patentblatt 88/44

(84) Benannte Vertragsstaaten: BE CH DE ES FR GB IT LI NL

(71) Anmelder: BAYER AG
Konzernverwaltung RP Patentabteilung
D-5090 Leverkusen 1 Bayerwerk(DE)

(72) Erfinder: Wollweber, Hartmund, Dr.
In den Birken 73
D-5600 Wuppertal 1(DE)
Erfinder: Stoltefuss, Jürgen, Dr.
Parkstrasse 20
D-5657 Haan 2(DE)
Erfinder: Berschauer, Friedrich, Dr.
Claudiusweg 9
D-5600 Wuppertal 1(DE)

## (54) Neue Aminophenylethylamin-Derivate, Verfahren zu ihrer Herstellung sowie ihre Verwendung als Leistungsförderer.

(57) Die vorliegende Erfindung betrifft neue Aminophenylethylamin-Derivate der allgemeinen Formeln

EP 0 288 867 A2

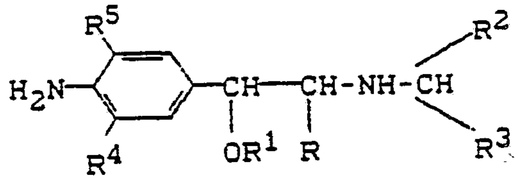

(I)

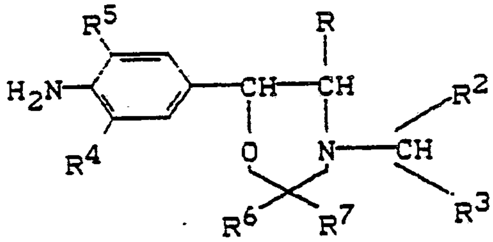

(Ia)

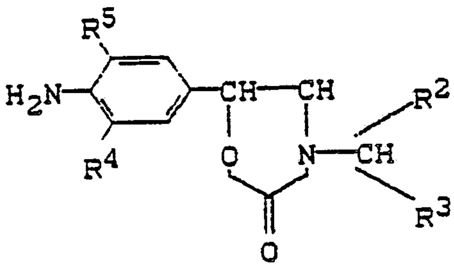

(Ib)

in welchen die Reste R, $R^1$ - $R^5$, die in der Beschreibung angegebenen Bedeutungen besitzen und Verfahren zu ihrer Herstellung gefunden.

Sie besitzen ausgezeichnete leistungsfördernde, insbesondere wachstumsfördernde Wirkung bei Tieren, z. B. bewirken sie eine Verschiebung des Fleich/Fett-Verhältnis zugunsten von Fleisch.

## Neue Aminophenylethylamin-Derivate, Verfahren zu ihrer Herstellung sowie ihre Verwendung als Leistungsförderer

Die vorliegende Erfindung betrifft neue Aminophenylethylamin-Derivate, Verfahren zu ihrer Herstellung und ihre Verwendung als Leistungsförderer für Tiere.

Die Verwendung von Futtermittelzusätzen zur Erzielung höherer Gewichtszuwächse und verbesserter Futterausnutzung wird in der Tierernährung, insbesondere bei der Mast von Schweinen, Rindern und Geflügel, bereits weitgehend praktiziert. Es ist bekannt, daß substituierte Arylethanolamine die Gewichtszunahme von Tieren fördern (EP-OS 26 298). Ihre Wirkung befriedigt jedoch nicht in allen Fällen.

Es wurden die neuen Verbindungen der allgemeinen Formel

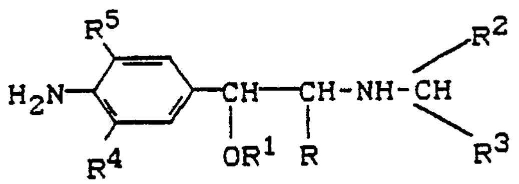

(I)

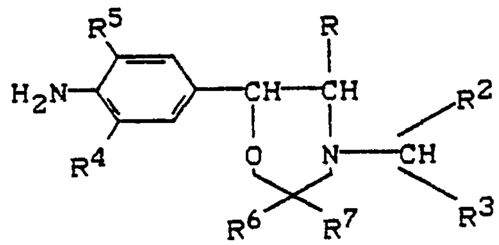

(Ia)

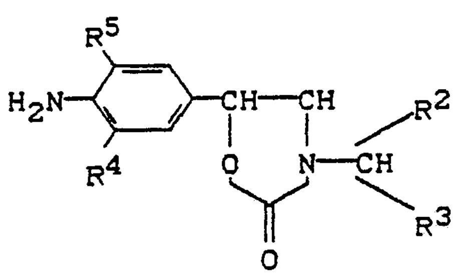

(Ib)

in der

R = H, Alkyl,

$R^1$ = Wasserstoff, Acyl, Aroyl, Arylacyl,

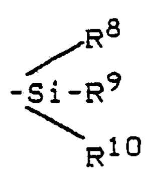

$R^2$ = Methyl oder Ethyl,

$R^3$ = durch Alkoxy, Alkylthio substituiertes geradkettiges, verzweigtes oder cyclisches Alkyl oder gegebenenfalls durch Alkyl substituiertes Heterocyclyl mit 4-6 Ringatomen und Sauerstoff als Heteroatom,

$R^4$ = H, Alkyl ($C_1$-$C_3$), Halogen, Trifluormethyl, CN,

$R^5$ = Alkyl ($C_1$-$C_3$), Halogen, Trifluormethyl, CN, Alkoxy, Alkylthio,

$R^6$ = H, Alkyl,

$R^7$ = H, Alkyl, Phenyl, Phenylalkyl,

und
$R^8$, $R^9$, $R^{10}$ = einen geradkettigen oder verzweigten Alkylrest bedeuten,
und ihre physiologisch verträglichen Salze gefunden. Die erfindungsgemäßen Verbindungen können in Form ihrer Racemate sowie Enantiomere oder Diastereomere vorliegen.

Sie besitzen ausgezeichnete leistungsfördernde, insbesondere wachstumsfördernde Wirkung bei Tieren, z.B. bewirken sie eine Verschiebung des Fleisch/Fett-Verhältnisses zugunsten von Fleisch.

Bevorzugte Verbindungen sind Phenylethylamin-Derivate der allgemeinen Formel (I),
in welcher
R = Wasserstoff oder Methyl und
$R^1$ = Wasserstoff oder die Reste $-Si(CH_3)_2CH(CH_3)CH(CH_3)_2$, $Si(CH_3)_2C(CH_3)_3$,
$R^2$ = Methyl
$R^3$ für den Rest $(CR^{11}R^{12})_n$-X-$R^{13}$ steht, wobei $R^{11}$ und $R^{12}$ für Wasserstoff oder Methyl stehen oder für $C_{5-6}$-Cycloalkyl das durch $C_{1-4}$-Alkoxy substituiert ist steht,
n = für 1, 2, 3 steht,
$R^{13}$ für $C_{1-4}$-Alkyl steht oder gemeinsam mit einem der Reste $R^{11}$ oder $R^{12}$ und den Atomen an die sie gebunden sind einen 4-6 gliedrigen Ring bildet der durch O unterbrochen ist und gegebenenfalls durch Methyl substituiert ist.

Ganz besonders bevorzugt sind Verbindungen der Formel I in welcher
der Rest

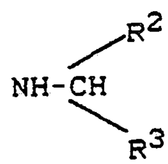

für folgende Reste steht:

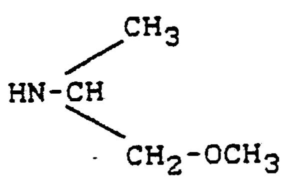 , 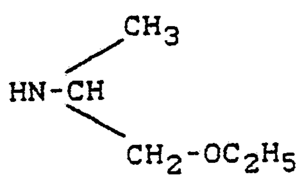 , 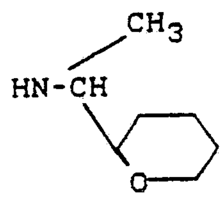 ,

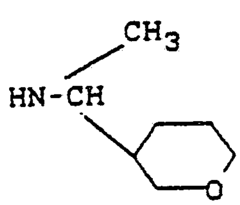 , 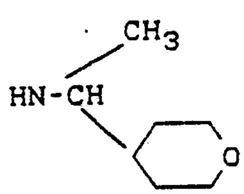 , 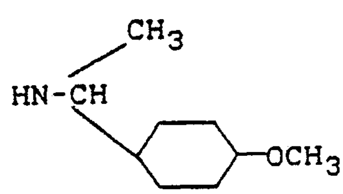 ,

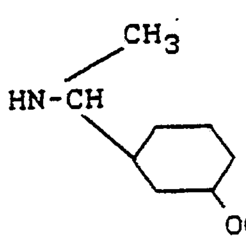 , 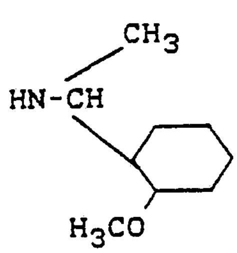 , 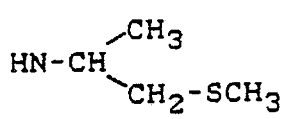 .

Man erhält die neuen Verbindungen der Formel (I)

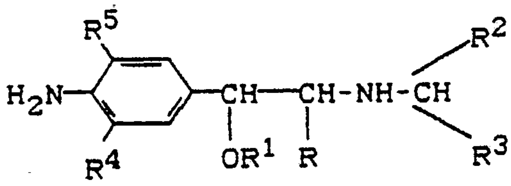

(I)

in welcher
R, $R^2$ bis $R^5$ die oben angegebenen Bedeutungen haben und
$R^1$ für Wasserstoff steht,
indem man
a) Verbindungen der Formel (II)

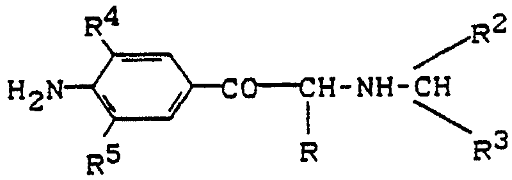

(II)

in welcher
R, $R^2$ bis $R^5$ die oben angegebenen Bedeutungen haben
katalytisch oder mit geeigneten Reduktionsmitteln wie z. B. Natriumborhydrid, Natriumcyanoborhydrid usw. reduziert, oder
b) Verbindungen der Formel (III)

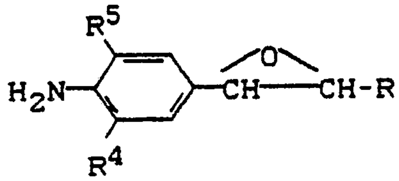

(III)

in welcher
R, $R^4$, $R^5$ die oben angegebenen Bedeutungen haben,
mit Aminen der Formel (IV)

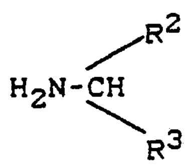

(IV)

umsetzt, in welcher
R, $R^2$, $R^3$ die oben angegebenen Bedeutungen haben,
oder
c) für den Fall von Verbindungen der Formel (I),
in welcher
R für Wasserstoff steht und
$R^1$ die oben angegebene Bedeutung hat,
indem man Verbindungen der Formel (V)

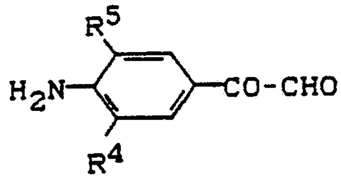

(V)

in welcher
$R^4$ und $R^5$ die oben angegebenen Bedeutungen haben,
mit einem Amin der Formel (IV) in Gegenwart eines Reduktionsmittels wie z. B. Natriumborhydrid umsetzt.

d) Man erhält die neuen Verbindungen der Formel (I),
in welcher
R, $R^2$ - $R^5$ die bei (I) angegebenen Bedeutungen haben und
$R^1$ für den Rest

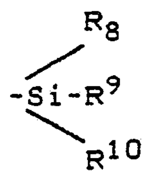

steht und
$R^8$, $R^9$, $R^{10}$ die oben angegebenen Bedeutungen haben,
indem man Verbindungen der Formel (I),
in welcher
$R^1$ für Wasserstoff steht,
mit geeigneten Silylierungsstoffen der Formel (VI)

$R^8$ $R^9$ $R^{10}$ Si Z (VI),

in der
Z für Halogen, CN, $OSO_2$-$CF_3$, -O-Si-$R^2R^3R^4$ oder -O-$SO_2$-O-Si-$R^8R^9R^{10}$ steht, und
$R^8$, $R^9$, $R^{10}$ die oben angegebenen Bedeutungen haben,
umsetzt, oder

e) die Verbindung der Formel (III) (oben) mit einem silylierten Amin der Formel (VII)

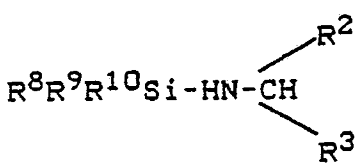

(VII)

in welcher
$R^8$, $R^9$, $R^{10}$ die oben angegebenen Bedeutungen haben,
umsetzt.

f) Man erhält die neuen Verbindungen der Formel (I), in welcher
R, $R^2$ - $R^5$ die bei (I) angegebenen Bedeutungen haben und
$R^1$ für Acyl, Aroyl, Arylacyl steht,
indem man die Verbindungen der Formel (I), in welcher
$R^1$ für Wasserstoff steht,
mit geeigneten acylierenden Mitteln umsetzt, oder

g) man erhält die Verbindungen der Formel (Ia), in welcher
$R^2$ - $R^7$ die bei (I) angegebenen Bedeutungen haben,
indem man die Verbindungen der Formel (I), in welcher $R^1$ für Wasserstoff steht,
mit Carbonylverbindungen der Formel

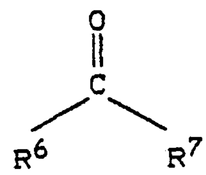

in welcher
$R^6$ und $R^7$ die oben angegebenen Bedeutungen haben,
gegebenenfalls in Gegenwart saurer Katalysatoren umsetzt, oder

h) man erhält Verbindungen der Formel (Ib), in welcher
$R^2$ - $R^5$ die oben angegebenen Bedeutungen haben,
indem man Verbindungen der Formel (I), in welcher
$R^1$ für Wasserstoff steht,
mit Kohlensäurederivaten, gegebenenfalls in Anwesenheit von Basen cyclisiert.

Die Ausgangsverbindungen der Formel (III) und (V) sind bereits bekannt (vgl. Z. B. R. E. Lutz and Coworkers, J. Org. Chem. 12, 617-703, DAS 23 54 959). Die Verbindungen der Formel (II) sind neu, können jedoch nach bekannte Methoden hergestellt werden (vgl. J. Org. Chem. 12, 617-703). Die Amine der Formel (IV) sind bekannt oder können nach bekannten Methoden durch Aminierung entsprechender Ketone hergestellt werden.

Die Verfahren a) - h) werden analog zu bekannten Methoden der organischen Chemie durchgeführt.

Die erfindungsgemäßen Verfahren a) - h) können in Gegenwart von Verdünnungsmittel durchgeführt werden. Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether, wie Diethyl-und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäure-methylester und -ethylester, ferner Nitrile, wie z. B. Acetonitril und Propionnitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Das erfindungsgemäße Verfahren d) kann in Gegenwart von Katalysatoren durchgeführt werden. Als Katalysatoren können bevorzugt verwendet werden: Imidazol, Triazol oder Diisopropylethylamin.

Die Reaktionstemperatur bei den Verfahren a) - h) wird zwischen etwa 0°C und 130°C, vorzugsweise zwischen etwa 20°C und 60°C, gehalten. Die Verfahren werden vorzugsweise bei Normaldruck durchgeführt.

Bei Verfahren e) werden die Ausgangsverbindungen der Formeln II und IV im allgemeinen in etwa äquimolarem Verhältnis eingesetzt, jedoch kann ein Überschuß bis zu 300 % der Verbindungen der Formel IV eingesetzt werden.

Bei den übrigen Verfahren werden die Ausgangsverbindungen im allgemeinen in etwa äquimolaren Verhältnis eingesetzt. Die Reduktionsmittel können in 1-bis 10-fachem Überschuß eingesetzt werden.

Die Wirkstoffe eignen sich bei günstiger Warmblütertoxizität als Mittel zur Leistungsförderung bei Zucht-und Nutztieren. Sie dienen dabei zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch.

Zu den Nutz-und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß-und Salzwasserfische wie z.B. Forellen, Karpfen, Aale.

Die Wirkstoffe werden unabhängig vom Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Darüberhinaus zeigen die erfindungsgemäßen Verbindungen gute antiinflammatorische Wirkung im Carrageenaninduzierten Pfotenödem der Ratte.

Sie können somit als Antiphlogistika, Antirheumatika sowie zur Behandlung von Entzündungen und Ödemen eingesetzt werden.

Besonders wertvoll erweisen sich die Wirkstoffe bei Aufzucht und Haltung von Jung-und Masttieren.

Die Anwendung der Wirkstoffe erfolgt direkt oder in Form von für Tiere geeigneten Zubereitungen

enteral oder parentereal. Die enterale Anwendung der Wirkstoffe geschieht z.B. oral in Form von Pulvern, Tabletten, Kapseln, Pasten, Tränken, Granulaten, oral applisierbaren Lösungen, Emulsionen oder Suspensionen, Boli sowie über das Futter oder über das Trinkwasser. Die parenterale Anwendung geschieht z.B. in Form der Injektion (intramuskulär, subcutan, intravenös oder durch Implantate).

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft, wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen nicht-Protein-Verbindungen, Farbstoffen, Antioxidantien, Aromastoffen, Emulgatoren, Fließhilfsstoffen, Konservierungs-und Preßhilfsstoffen vorliegen.

Andere leistungsfördernde Wirkstoffe sind z.B. Antibiotika wie Tylosin und Virginiamycin.
Mineralische Futtermittel sind z.B. Dicalciumphosphat, Magnesiumoxid, Natriumchlorid.
Spurenelement-Verbindungen sind z.B. Eisenfumarat, Natriumjodid, Kobaltchlorid, Kupfersulfat, Zinkoxid, Selenverbindungen.
Vitamine sind z.B. Vitamin A, Vitamin $D_3$, Vitamin E.

Stickstoffhaltige nicht-Protein-Verbindungen sind z.B. Biuret, Harnstoff.
Farbstoffe sind z.B. Carotinoide wie Canthaxidin, Zeaxanthin, Capsanthin, oder alle Farbstoffe, die zur Färbung von Lebensmitteln zugelassen sind.
Antioxidantien sind z.B. Ethoxyquin, Butylhydroxy-Toluol, Ascorbinsäure.
Aromastoffe sind z.B. Vanillin.

Emulgatoren sind z.B. Ester der Milchsäure, Lecithin. Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure.
Preßhilfsstoffe sind z.B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sich durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums-und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:
200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung der unten angegebenen Zusammensetzung und 2,5 g Wirkstoff-Prämix der unten angegebenen Zusammensetzung ergeben nach sorgfältigem Mischen 1 kg Futter.

In einem kg Vitamin-Mineral-Mischung sind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin

20 mcg Vitamin $B_{12}$, 5 mg Calciumpantothenat, 30 mg Niktoinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zumannengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix der unten angegebenen Zusammensetzung ergeben nach sorgfältigem Mischen 1 kg Futter.

1 kg Wirkstoff Prämix enthält 200 g Wirkstoff, 20 g Pflanzenöl und 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der Zusammensetzung wie beim Schweinefutter angegeben. Die Vitamin-Mineral-Mischung enthält pro kg 70.000 i.E. Vitamin A, 70.000 i:E. Vitamin $D_3$, 100 mg Vitamin E, 50 mg Mn $SO_4$ x $H_2O$, 30 mg Zn $SO_4$ x $7H_2O$ in Getreidemehl als Trägerstoff.

Der Wirkstoff-Prämix wird der Vitamin-Mineral-Mischung in der erforderlichen Mengen beigemischt und diese anschließend sorgfältig mit den übrigen Bestandteilen vermischt.

Beispiel A

Ratten-Fütterungsversuch

Weibliche Laborratten 90 - 110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichen der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Menge an perirenalen Fettgewebe bestimmt.

Es werden die aus der Tabelle ersichtlichen Ergebnisse erhalten:

Tabelle A

Ratten Fütterungsversuch

| Wirkstoff Beispiel | Dosis ppm | Tierzahl | Zuwachs (relativ zur Kontrolle) |
|---|---|---|---|
| Kontrolle | 25 | 12 | 100 |
| Seite 22 } Zeile 6 } | 25 | 12 | 118 |
| Seite 20 } Zeile 9 } | 25 | 12 | 126 |
| Seite 21 } Zeile 35 } | 25 | 12 | 125 |
| Seite 20 } Zeile 35 } | 25 | 12 | 132 |

Herstellungsbeispiele

Beispiel 1

2-(3,5-Dichlor-4-amino-phenyl)-2-hydroxy-N-(1-methyl-2-methoxy-ethyl)-ethylamin

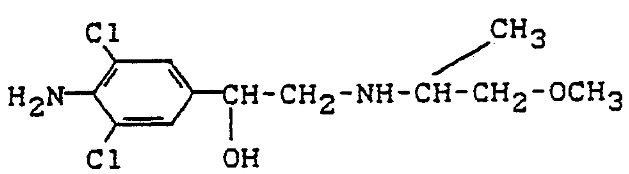

33 g 4-Amino-3,5-Dichloro-ω-(1-methyl-2-methoxyethylamino)-acetophenon-hydrochlorid werden in 180 ml Methanol und 83 ml Wasser gelöst und tropfenweise mit einer Lösung von 7,6 g Natriumborhydrid in 30 ml Wasser versetzt, wobei der pH-Wert durch Zugabe von ca. 10 %iger Salzsäure zwischen 3 und 7 gehalten wird. Nach beendeter Reaktion wird stark sauer gestellt und eingeengt. Es wird mit Ammoniak basisch gestellt, mit Essigester extrahiert, die Essigester-Phase zweimal mit Wasser gewaschen, getrocknet und eingeengt. Die erhaltenen Kristalle werden mit Heptan verrührt und abgesaugt. Man erhält 30,4 g farblose Kristalle vom Fp.: 90-95°C. (Diastereomerengemisch).

Durch Umlösen aus Essigester erhält man aus dem Diastereomerengemisch die reinen Diastereomeren A und B, die an Hand der Signale der Methoxygruppen im $^1$H-NMR Spektrum charakterisiert werden. Die in Chloroform konzentrationsabhängigen Signale liegen bei $\delta$ 3,3-3,4 ppm. Dem Diastereomer A, Fp. 111-112°C, wird das Signal (bei höheren Frequenzen/niederem Feld) zugerechnet.

Analog Beispiel 1 wurden hergestellt:

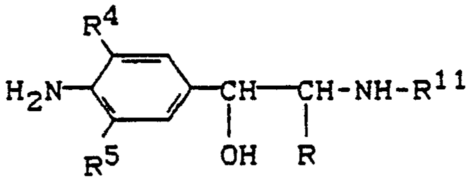

| R | $R^4$ | $R^5$ | $R^{11}$ | F [°C] |
|---|---|---|---|---|
| H | Cl | Cl | $CH(CH_3)CH_2-OC_2H_5$ | |
| H | Cl | Cl | $CH(CH_3)$ 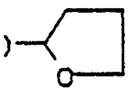 | 110-120 |
| H | Cl | Cl | $CH(CH_3)$ 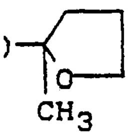 | 75- 80 |
| H | Cl | Cl | $CH(CH_3)$ 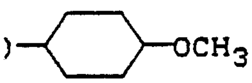 | |
| H | Cl | Cl | $CH(CH_3)$ 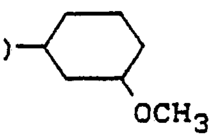 | |
| H | Cl | Cl | $CH(CH_3)$ 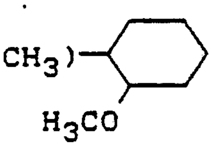 | |
| H | Cl | Cl | $CH(CH_3)$ 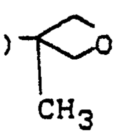 | |
| H | Cl | Cl | $CH(CH_3)$ 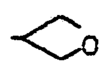 | |
| H | Cl | Cl | $CH(CH_3)$ 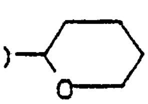 | Öl |

| R | $R^4$ | $R^5$ | $R^{11}$ | F [°C] |
|---|---|---|---|---|
| H | Cl | Cl | $CH(CH_3)-$ 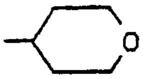 | 133 |
| H | Cl | Cl | $CH(CH_3)-$ 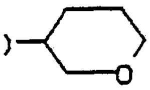 | Öl |
| $CH_3$ | Cl | Cl | $CH(CH_3)CH_2-OCH_3$ | |
| H | Cl | $CF_3$ | $CH(CH_3)CH_2-OCH_3$ | |
| H | Cl | $CF_3$ | $CH(CH_3)-$ 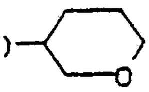 | |
| H | Cl | $CF_3$ | $CH(CH_3)-$ 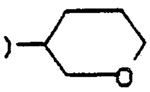 | |
| H | Cl | $CF_3$ | $CH(CH_3)-$ 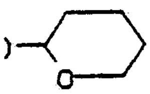 | |
| H | Cl | $CF_3$ | $CH(CH_3)-$ 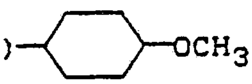 | |
| H | Cl | H | $CH(CH_3)CH_2-OCH_3$ | |
| H | Cl | H | $CH(CH_3)-$ 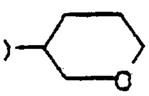 | |
| H | Cl | H | $CH(CH_3)-$ 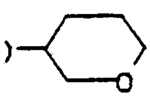 | |
| H | Cl | H | $CH(CH_3)-$ 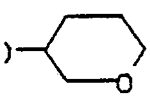 | |
| H | Cl | H | $CH(CH_3)-$ 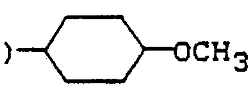 | |

| R | $R^4$ | $R^5$ | $R^{11}$ |
|---|---|---|---|
| H | CN | H | $CH(CH_3)CH_2-OCH_3$ |
| H | CN | H | $CH(CH_3)-$ 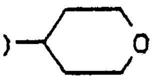 |
| H | CN | H | $CH(CH_3)-$ 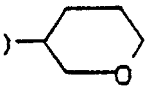 |
| H | CN | H | $CH(CH_3)-$ 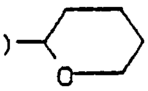 |
| H | CN | H | $CH(CH_3)-$ 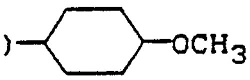 |
| H | Cl | $CH_3$ | $CH(CH_3)CH_2-OCH_3$ |
| H | Cl | $CH_3$ | $CH(CH_3)-$ 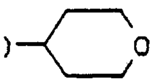 |
| H | Cl | $CH_3$ | $CH(CH_3)-$ 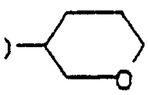 |
| H | Cl | $CH_3$ | $CH(CH_3)-$ 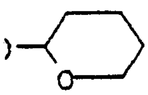 |
| H | Cl | $CH_3$ | $CH(CH_3)-$ 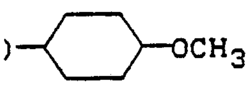 |
| H | $CH_3$ | $CH_3$ | $CH(CH_3)CH_2-OCH_3$ |
| H | $CH_3$ | $CH_3$ | $CH(CH_3)-$ 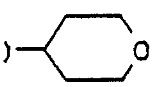 |
| H | $CH_3$ | $CH_3$ | $CH(CH_3)-$ 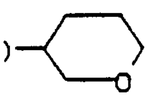 |

| R | $R^4$ | $R^5$ | $R^{11}$ | |
|---|---|---|---|---|
| H | $CH_3$ | $CH_3$ | $CH(CH_3)$- 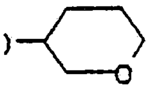 | |
| H | $CH_3$ | $CH_3$ | $CH(CH_3)$- 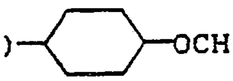 $OCH_3$ | |
| H | Cl | F | $CH(CH_3)CH_2$-$OCH_3$ | |
| H | Cl | F | $CH(CH_3)$- 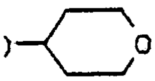 | |
| H | Cl | F | $CH(CH_3)$- 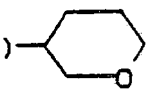 | |
| H | Cl | F | $CH(CH_3)$- 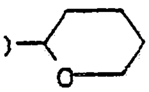 | |
| H | Cl | F | $CH(CH_3)$- 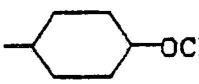 $OCH_3$ | |
| H | $CH_3$ | H | $CH(CH_3)CH_2$-$OCH_3$ | |
| H | $CH_3$ | H | $CH(CH_3)$- 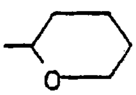 | |
| H | Cl | Cl | $CH(CH_3)$-$C(CH_3)_2$-O-$CH_3$ | 118-120° C |
| H | $CH_3$ | H | $CH(CH_3)$- 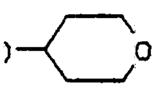 | |
| H | $H_3CHO$ | $CH_3$ | $CH(CH_3)$-$CH_2$-$OCH_3$ | |
| H | $H_3CO$ | H | $CH(CH_3)$- 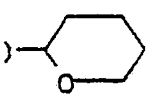 | |
| H | $H_3CO$ | H | $CH(CH_3)$- 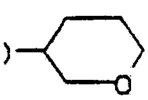 | |
| H | Cl | Cl | $CH(CH_3)$-$CH_2$-$SCH_3$ | |

Beispiel 2

2-(3,5-Dichlor-4-amino-phenyl)-2-(2,2-dimethylpropyl-dimethylsilyloxy)-N-(1-methyl-2-methoxyethyl)-ethylamin

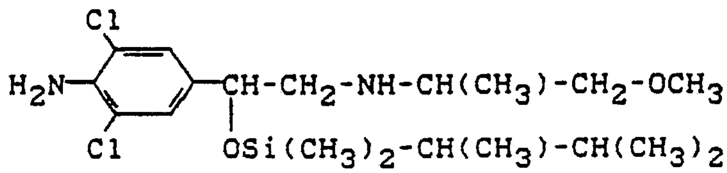

1,36 g (0,02 mol) Imidazol werden in 15 ml abs. DMF mit 2,93 g (0,01 mol) der Verbindung vom Beispiel 1 versetzt. Bei 0 bis 5°C werden nun 1,8 g Dimethyl-1,2-dimethyl-propyl-silylchlorid zugegeben. Es wird 2 Stunden unter Eiskühlung gerührt, eingeengt, in Toluol/Wasser aufgenommen, getrennt, die Toluolphase 4 x mit Wasser gewaschen, getrocknet, eingeengt und an der Ölpumpe von Lösungsmittelresten befreit. Man erhält 4,3 g eines nahezu farblosen Öles.

In entsprechender Arbeitsweise werden folgende Silyloxyverbindungen hergestellt:

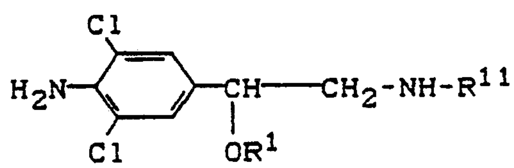

| $R^1$ | $R^{11}$ |
|---|---|
| $Si(CH_3)_3$ | $CH(CH_3)CH_2OCH_3$ |
| $Si(CH_3)_2-C(CH_3)_3$ | $CH(CH_3)CH_2OCH_3$ |

| $R^{10}$ | $R^{11}$ | |
|---|---|---|
| $Si(CH_3)_2-CH(CH_3)-CH(CH_3)_2$ | $CH(CH_3)CH_2OC_2H_5$ | |
| $Si(CH_3)_3$ | $CH(CH_3)CH_2OC_2H_5$ | |
| $Si(CH_3)_2-C(CH_3)_3$ | $CH(CH_3)CH_2OC_2H_5$ | |
| $Si(CH_3)_2-CH(CH_3)-CH(CH_3)_2$ | $CH(CH_3)$ 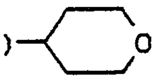 | |
| $Si(CH_3)_3$ | $CH(CH_3)$ 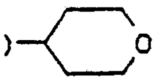 | |
| $Si(CH_3)_2-C(CH_3)_3$ | $CH(CH_3)$ 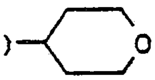 | |
| $Si(CH_3)_2-CH(CH_3)-CH(CH_3)_2$ | $CH(CH_3)$ 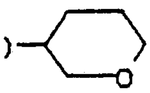 | |
| $Si(CH_3)_3$ | $CH(CH_3)$ 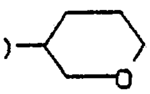 | |
| $Si(CH_3)_2-C(CH_3)_3$ | $CH(CH_3)$ 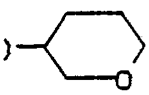 | |
| $Si(CH_3)_2-CH(CH_3)-CH(CH_3)_2$ | $CH(CH_3)$ 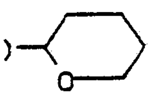 | Öl |
| $Si(CH_3)_3$ | $CH(CH_3)$ 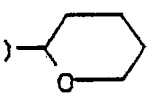 | |
| $Si(CH_3)_2-C(CH_3)_3$ | $CH(CH_3)$ 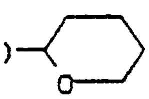 | |
| $Si(CH_3)_2-CH(CH_3)-CH(CH_3)_2$ | $CH(CH_3)$ 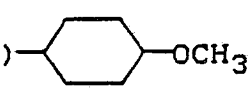 | |

| $R^{10}$ | $R^{11}$ |
|---|---|
| $Si(CH_3)_3$ | 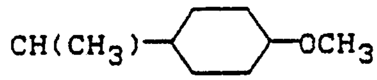 |
| $Si(CH_3)_2-C(CH_3)_3$ | 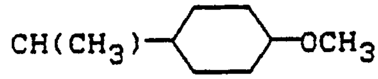 |
| $Si(CH_3)_2-CH(CH_3)-CH(CH_3)_2$ | 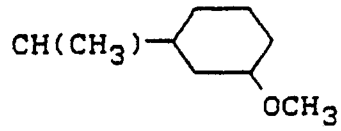 |
| $Si(CH_3)_3$ | 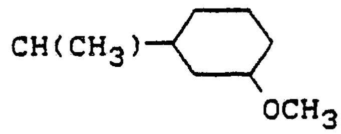 |
| $Si(CH_3)_2-C(CH_3)_3$ | 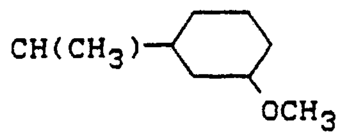 |

Beispiel 3

2-(3,5-Dichlor-4-amino-phenyl)-2-acetoxy-N-(1-methyl-2-methoxy-ethyl)-ethylamin

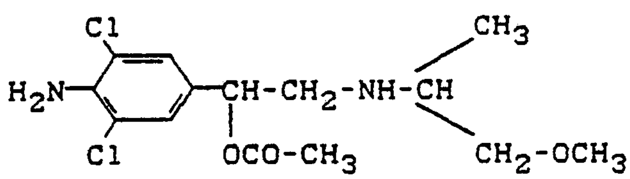

Zu 2,93 g (0,01 mol) 2-(3,5-Dichlor-4-amino-phenyl)-2-hydroxy-N-(1-methoxy-ethyl)-ethylamin (Beispiel 1) gelöst in 50 ml $CHCl_3$ fügt man 3 g Triethylamin, tropft 1,1 g (0,011 mol) Acetanhydrid bei -5°C zu und erwärmt 2 Stunden auf Zimmertemperatur. Nach beendeter Umsetzung (Kontrolle durch DC) dampft man ein im Vakuum, nimmt in Essigester auf, wäscht mit Natriumbicarbonatlösung, dann mit gesättigter Kochsalzlösung, dampft die organische Phase ein und chromatographiert über eine Kieselgelsäule (Hexan-Essigester = 2:1). Ausbeute: 2,5 g.

In entsprechender Arbeitsweise erhält man folgende Ester:

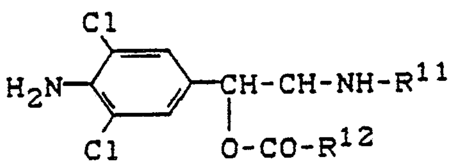

| $R^{11}$ | $R^{12}$ |
|---|---|
| $C_2H_5$ | $CH(CH_3)CH_2OCH_3$ |
| $CH(CH_3)_2$ | $CH(CH_3)CH_2OCH_3$ |
| $C_4H_9$ | $CH(CH_3)CH_2OCH_3$ |
| $C_6H_5$ | $CH(CH_3)CH_2OCH_3$ |
| $CH_2C_6H_5$ | $CH(CH_3)CH_2OCH_3$ |
| $CH_3$ | $CH(CH_3)CH_2OC_2H_5$ |
| $C_2H_5$ | $CH(CH_3)CH_2OC_2H_5$ |
| $CH(CH_3)_2$ | $CH(CH_3)CH_2OC_2H_5$ |
| $C_4H_9$ | $CH(CH_3)CH_2OC_2H_5$ |
| $C_6H_5$ | $CH(CH_3)CH_2OC_2H_5$ |
| $CH_2C_6H_5$ | $CH(CH_3)CH_2OC_2H_5$ |
| $CH_3$ | $CH(CH_3)$ 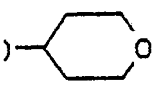 |

| $R^{11}$ | $R^{12}$ |
|---|---|
| $C_2H_5$ | $CH(CH_3)$ 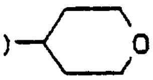 |
| $CH(CH_3)_2$ | $CH(CH_3)$ 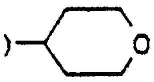 |
| $C_4H_9$ | $CH(CH_3)$ 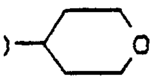 |
| $C_6H_5$ | $CH(CH_3)$ 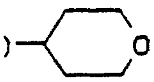 |
| $CH_2C_6H_5$ | $CH(CH_3)$ 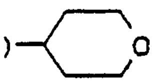 |
| $CH_3$ | $CH(CH_3)$ 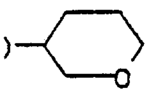 |
| $C_2H_5$ | $CH(CH_3)$ 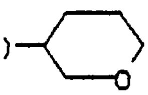 |
| $CH(CH_3)_2$ | $CH(CH_3)$ 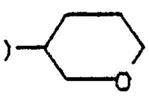 |
| $C_4H_9$ | $CH(CH_3)$ 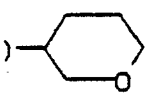 |
| $C_6H_5$ | $CH(CH_3)$ 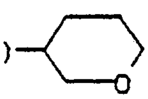 |
| $CH_2C_6H_5$ | $CH(CH_3)$ 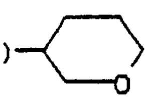 |

| $R^{11}$ | $R^{12}$ |
|---|---|
| $CH_3$ | $CH(CH_3)$- 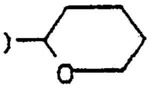 |
| $C_2H_5$ | $CH(CH_3)$- 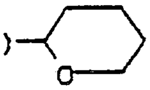 |
| $CH(CH_3)_2$ | $CH(CH_3)$- 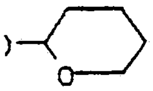 |
| $C_4H_9$ | $CH(CH_3)$- 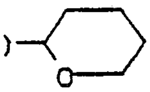 |
| $C_6H_5$ | $CH(CH_3)$- 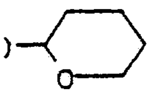 |
| $CH_2C_6H_5$ | $CH(CH_3)$- 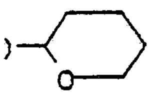 |
| $CH_3$ | $CH(CH_3)$- 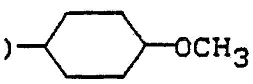 |
| $C_2H_5$ | $CH(CH_3)$- 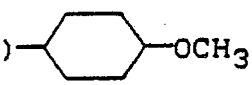 |
| $CH(CH_3)_2$ | $CH(CH_3)$- 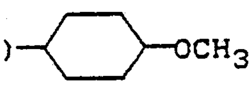 |
| $C_4H_9$ | $CH(CH_3)$- 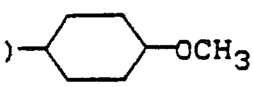 |
| $C_6H_5$ | $CH(CH_3)$- 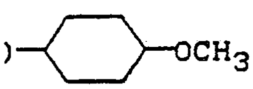 |

| $R^{11}$ | $R^{12}$ |
|---|---|
| $CH_2C_6H_5$ | 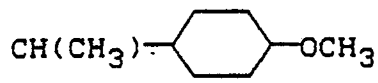 |
| $CH_3$ | 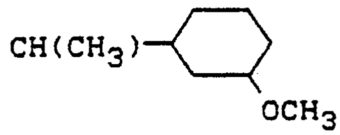 |
| $C_2H_5H_3)_2$ | 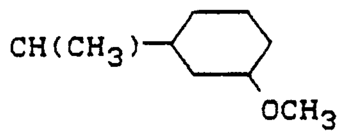 |
| $CH(CH_3)_2$ | 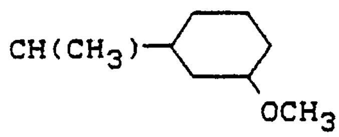 |
| $C_4H_9$ | 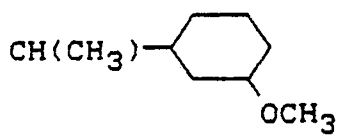 |
| $C_6H_5$ | 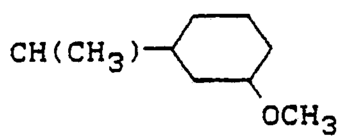 |
| $CH_2C_6H_5$ | 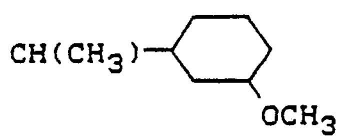 |

Beispiel 4

5-(3,5-Dichlor-4-amino-phenyl)-3-(1-methyl-2-methoxy-ethyl)-2-oxazolidinon

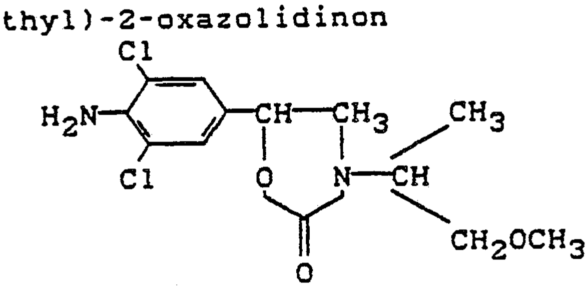

Zu 2,93 g (0,01 mol) 2-(3-5-Dichlor-4-amino-phenyl)-2-hydroxy-N-(1-methyl-2-methoxyethyl)-ethylamin, gelöst in 30 ml Methylenchlorid und 3 g Triethylamin tropft man bei -5°C eine Lösung von 1 g $COCl_2$ in 20 ml Methylenchlorid innerhalb von 20 Minuten. Man rührt 30 Minuten bei -5°C und 1 Stunde bei Raumtemperatur, dampft ein im Vakuum und chromatographiert den Rückstand über ein Kieselgelsäule n-Heptan-$CH_2Cl_2$ = 1 : 1.
Ausbeute: 1,3 g.
In entsprechender Weise erhält man folgende Oxazolidinone:

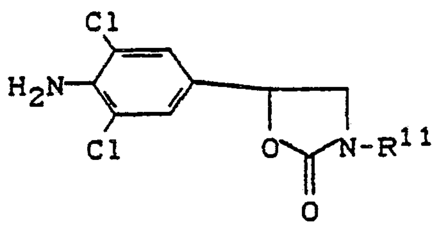

| $R_{11}$ |
|---|
| $CH(CH_3)-CH_2OC_2H_5$ |
| 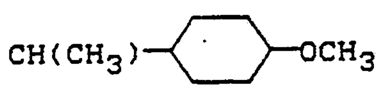 |
| 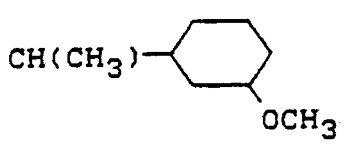 |
| 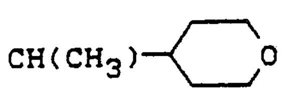 |
| 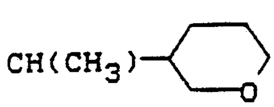 |
| 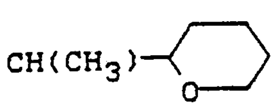 |

Beispiel 5

2-(3,5-Dichlor-4-amino-phenyl)-3-(1-methyl-2-methoxy-ethyl)-2-oxazolidinon

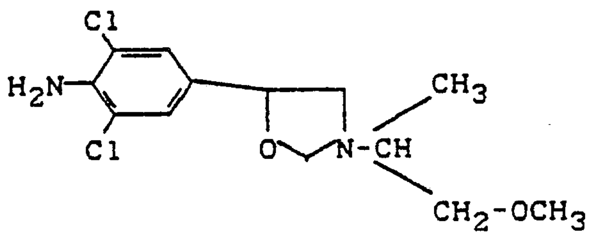

2,93 g (0,01 mol) 2-(3,5-Dichlor-4-amino-phenyl)-2-hydroxy-N-(1-methyl-2-methoxy-ethyl)-ethylamin, gelöst in 60 ml Toluol erhitzt man mit 100 ml Formaldehyde (38 %ig) unter Zusatz von 2 mg p-Toluolsulfonsäure am Wasserabscheider, wenn sich kein Wasser mehr abscheidet (ca. 1 Stunde) kühlt man ab. wäscht 2 mal mit 10%iger Natronlauge, dann mit Wasser und trocknet über $Na_2SO_4$. Nach dem Abdampfen chromatographiert man über eine Kieselgelsäule (Hexan, Methylenchlorid = 1 : 1) und erhält 2,4 g eines Öls.

In entsprechender Weise erhält man folgende Oxazolidine:

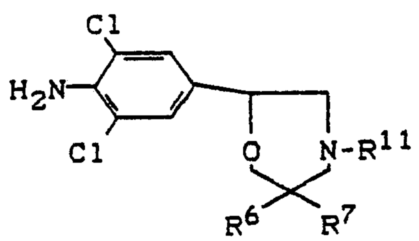

| $R^{11}$ | $R^6$ | $R^7$ |
|---|---|---|
| $CH(CH_3)CH_2OCH_3$ | H | $CH_3$ |
| $CH(CH_3)CH_3OCH_3$ | H | $C_6H_5$ |

| $R^{11}$ | $R^6$ | $R^7$ |
|---|---|---|
| $CH(CH_3)CH_2OCH_3$ | $CH_3$ | $CH_3$ |
| $CH(CH_3)CH_3OCH_3$ | $CH_3$ | $C_6H_5$ |
| $CH(CH_3$ 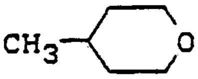 | H | H |
| $CH(CH_3)$ 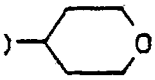 | H | $CH_3$ |
| $CH(CH_3)$ 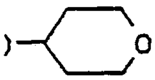 | H | $C_6H_5$ |
| $CH(CH_3)$ 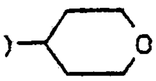 | $CH_3$ | $CH_3$ |
| $CH(CH_3)$ 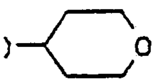 | $CH_3$ | $C_6H_5$ |
| $CH(CH_3)$ 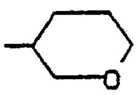 | H | H |
| $CH(CH_3)$ 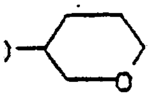 | H | $CH_3$ |
| $CH(CH_3)$ 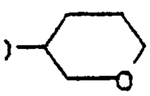 | H | $C_6H_5$ |
| $CH(CH_3)$ 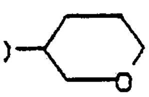 | $CH_3$ | $CH_3$ |
| $CH(CH_3)$ 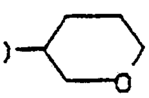 | $CH_3$ | $C_6H_5$ |

| $R^{11}$ | $R^6$ | $R^7$ |
|---|---|---|
| 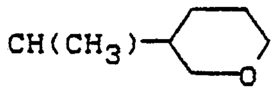 | H | H |
| 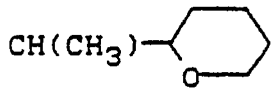 | H | $CH_3$ |
| 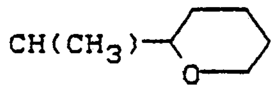 | H | $C_6H_5$ |
| 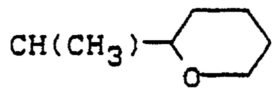 | $CH_3$ | $CH_3$ |
| 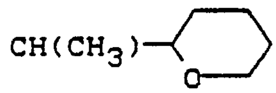 | $CH_3$ | $C_6H_5$ |
| 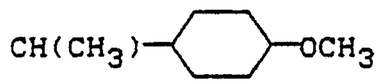 | H | H |
| 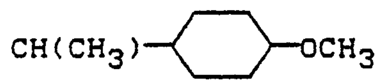 | H | $CH_3$ |
| 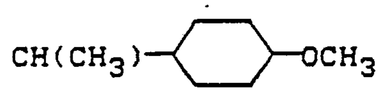 | H | $C_6H_5$ |
| 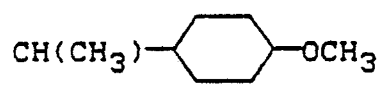 | $CH_3$ | $CH_3$ |
| 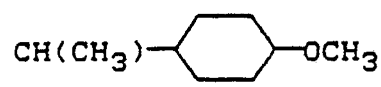 | $CH_3$ | $C_6H_5$ |
| 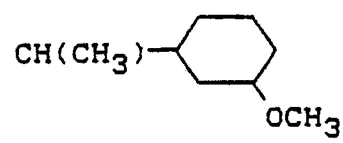 | H | H |

| $R^{11}$ | $R^6$ | $R^7$ |
|---|---|---|
| 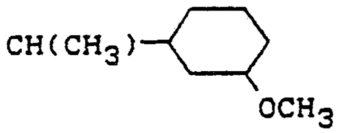 | H | $CH_3$ |
| 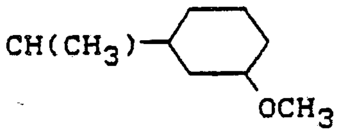 | H | $C_6H_5$ |
| 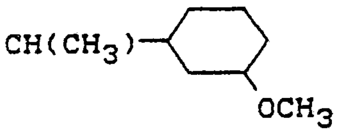 | $CH_3$ | $CH_3$ |
| 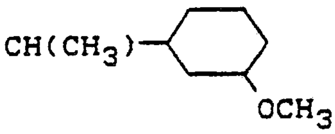 | $CH_3$ | $C_6H_5$ |
| 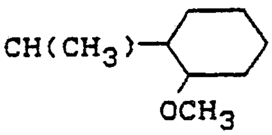 | H | H |
| 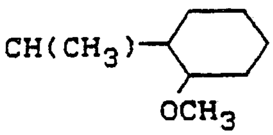 | H | $CH_3$ |
| 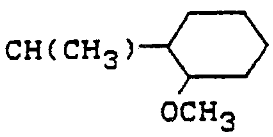 | H | $C_6H_5$ |
| 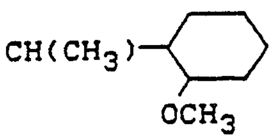 | $CH_3$ | $CH_3$ |
| 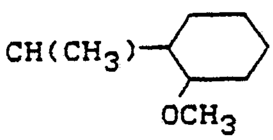 | $CH_3$ | $C_6H_5$ |

## Ansprüche

1. Aminophenylethylamin-Derivate der allgemeinen Formeln

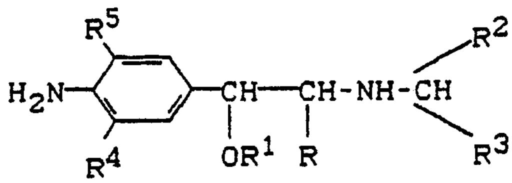

(I)

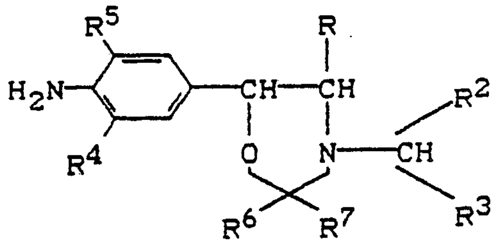

(Ia)

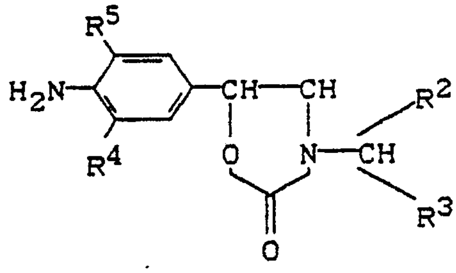

(Ib)

in welchen
R = H, Alkyl,
$R^1$ = Wasserstoff, Acyl, Aroyl, Arylacyl,

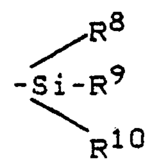

$R^2$ = Methyl oder Ethyl,
$R^3$ = durch Alkoxy, Alkylthio substituiertes geradkettiges verzweigtes oder cyclisches Alkyl oder gegebenenfalls durch Alkyl substituiertes Heterocyclyl mit 4-6 Ringatomen und 0 als Heteroatom,
$R^4$ = H, Alkyl ($C_1$-$C_3$), Halogen, Trifluormethyl, CN,
$R^5$ = Alkyl ($C_1$-$C_3$), Halogen, Trifluormethyl, CN, Alkoxy, Alkylthio,
$R^6$ = H, Alkyl,
$R^7$ = H, Alkyl, Phenyl, Phenylalkyl,
und
$R^8$, $R^9$, $R^{10}$ = einen geradkettigen oder verzweigten Alkylrest bedeuten.

2. Verfahren zur Herstellung der Aminophenylethylamin-Derivate der allgemeinen Formeln

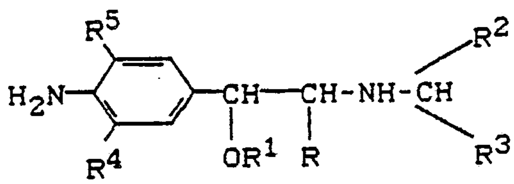

(I)

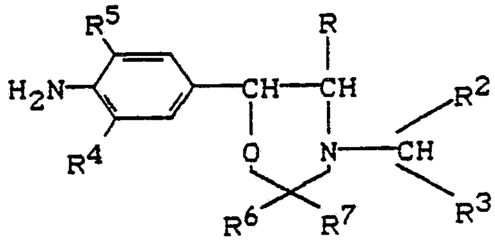

(Ia)

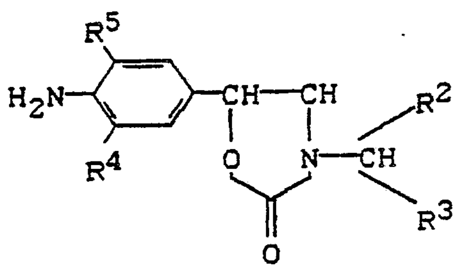

(Ib)

in welchen
R = H, Alkyl,
$R^1$ = Wasserstoff, Acyl, Aroyl, Arylacyl,

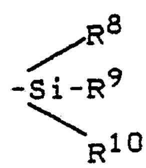

$R^2$ = Methyl oder Ethyl,
$R^3$ = durch Alkoxy, Alkylthio substituiertes geradkettiges verzweigtes oder cyclisches Alkyl oder gegebenenfalls durch Alkyl substituiertes Heterocyclyl mit 4-6 Ringatomen und 0 als Heteroatom,
$R^4$ = H, Alkyl ($C_1$-$C_3$), Halogen, Trifluormethyl, CN,
$R^5$ = Alkyl ($C_1$-$C_3$), Halogen, Trifluormethyl, CN, Alkoxy, Alkylthio,
$R^6$ = H, Alkyl,
$R^7$ = H, Alkyl, Phenyl, Phenylalkyl,
und
$R^8$, $R^9$, $R^{10}$ = einen geradkettigen oder verzweigten Alkylrest bedeuten,
dadurch gekennzeichnet, daß für den Fall der Verbindungen der Formel (I)

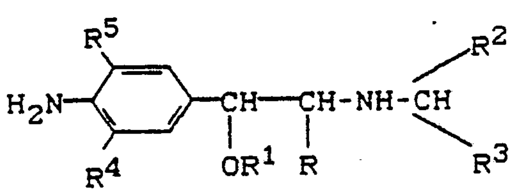

(I)

in welcher
R, $R^2$ bis $R^5$ die oben angegebenen Bedeutungen haben und
$R^1$ = Wasserstoff bedeutet,

a) Verbindungen der Formel (II)

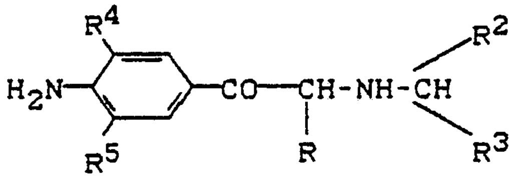

(II)

in welcher
R, $R^2$ bis $R^5$ die oben angegebenen Bedeutungen haben
katalytisch oder mit geeigneten Reduktionsmitteln reduziert werden oder

b) Verbindungen der Formel (III)

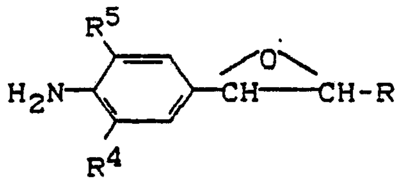

(III)

in welcher
R, $R^4$, $R^5$ die oben angegebenen Bedeutungen haben,
mit Aminen der Formel (IV)

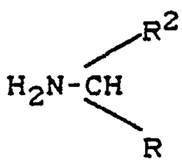

(IV)

umgesetzt werden oder

c) für den Fall von Verbindungen der Formel (I),
in welcher
R für Wasserstoff steht und
$R^1$ die oben angegebene Bedeutung hat,
Verbindungen der Formel (V)

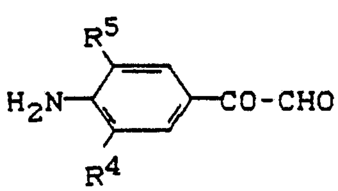

(V)

mit einem Amin der Formel (IV) (oben) in Gegenwart eines Reduktionsmittels wie z. B. Natriumborhydrid umsetzt werden oder

d) für den Fall von Verbindungen der Formel (I),
in welcher
R, $R^2$ - $R^5$ die oben angegebenen Bedeutungen haben und
$R^1$ für den Rest

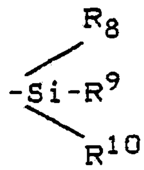

steht und
$R^8$, $R^9$, $R^{10}$ die oben angegebenen Bedeutungen haben,
Verbindungen der Formel (I),
in welcher
$R^1$ für Wasserstoff steht,
mit geeigneten Silylierungsstoffen der Formel (VI)

$R^8\ R^9\ R^{10}\ Si\ Z$ (VI),

in der
Z Halogen, CN, $OSO_2$-$CF_3$, -O-Si-$R^2R^3R^4$ oder -O-$SO_2$-O-Si-$R^8R^9R^{10}$ bedeutet, und
$R^8$, $R^9$, $R^{10}$ die oben angegebenen Bedeutungen haben,
umgesetzt werden oder

e) Verbindungen der Formel (III) (oben) mit einem silylierten Amin der Formel (VII)

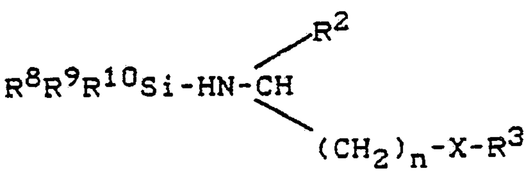 (VII)

in welcher
$R^8$, $R^9$, $R^{10}$ die oben angegebenen Bedeutungen haben,
umgesetzt werden oder

f) für den Fall von Verbindungen der Formel (I), in welcher
R, $R^2$ - $R^5$ die bei (I) angegebenen Bedeutungen haben und
$R^1$ für Acyl, Aroyl, Arylacyl steht,
Verbindungen der Formel (I), in welcher
$R^1$ für Wasserstoff steht,
mit acylierenden Mitteln umgesetzt werden, oder

g) für den Fall der Verbindungen der Formel (Ia), in welcher
$R^2$ - $R^7$ die oben angegebenen Bedeutungen haben,
Verbindungen der Formel (I), in welcher $R^1$ für Wasserstoff steht,
mit Carbonylverbindungen der Formel

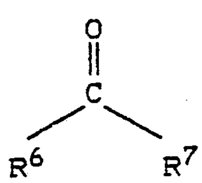

in welcher
$R^6$ und $R^7$ die oben angegebenen Bedeutungen haben,
gegebenenfalls in Gegenwart saurer Katalysatoren umgesetzt werden, oder

h) für den Fall von Verbindungen der Formel (Ib), in welcher
$R^2$ - $R^5$ die oben angegebenen Bedeutungen haben,
Verbindungen der Formel (I), in welcher
$R^1$ für Wasserstoff steht,
mit Kohlensäurederivaten, gegebenenfalls in Anwesenheit von Basen cyclisiert werden.

3. Mittel zur Leistungsförderung von Tieren, gekennzeichnet durch einen Gehalt an Aminophenylethylamin-Derivaten der Formeln I, Ia und Ib gemäß Anspruch 1.

4. Tierfutter, Trinkwasser für Tiere, Zusätze für Tierfutter und Trinkwasser für Tiere, gekennzeichnet durch einen Gehalt an Aminophenylethylamin-Derivaten der Formeln I, Ia und Ib gemäß Anspruch 1.

5. Verwendung von Aminophenylethylamin-Derivaten der Formeln I, Ia und Ib gemäß Anspruch 1 zur Leistungsförderung von Tieren.

6. Verfahren zur Herstellung von Mitteln zur Leistungsförderung von Tieren, dadurch gekennzeichnet, daß man Aminophenylethylamin-Derivate der Formeln I, Ia und Ib gemäß Anspruch 1 mit Streck-und/oder Verdünnungsmitteln vermischt.

7. Verfahren zur Herstellung von Tierfutter, Trinkwasser für Tiere oder Zusätze für Tierfutter und Trinkwasser für Tiere, dadurch gekennzeichnet, daß man Aminophenylethylamin-Derivate der Formel I, Ia und Ib gemäß Anspruch 1 mit Futtermitteln oder Trinkwasser und gegebenenfalls weiteren Hilfsstoffen vermischt.

8. Verwendung von Aminophenylethylamin-Derivaten der Formeln I, Ia und Ib gemäß Anspruch 1 zur Herstellung von Mitteln zur Leistungsförderung bei Tieren.